**Europäisches Patentamt**

**European Patent Office**

**Office européen des brevets**

(19)

(11) Numéro de publication: **0 318 342 B1**

(12) **FASCICULE DE BREVET EUROPEEN**

(45) Date de publication du fascicule du brevet:
28.11.90

(51) Int. Cl.⁵: **C01B 3/32**

(21) Numéro de dépôt: **88402682.4**

(22) Date de dépôt: **25.10.88**

(54) Procédé de reformage de méthanol impur et dispositif de mise en oeuvre.

(30) Priorité: **29.10.87 FR 8715006**

(43) Date de publication de la demande:
**31.05.89 Bulletin 89/22**

(45) Mention de la délivrance du brevet:
**28.11.90 Bulletin 90/48**

(84) Etats contractants désignés:
**DE FR GB**

(56) Documents cités:
**FR-A- 2 490 615**
**FR-A- 2 572 380**

(73) Titulaire: **L'AIR LIQUIDE, SOCIETE ANONYME POUR L'ETUDE ET L'EXPLOITATION DES PROCEDES GEORGES CLAUDE, 75, Quai d'Orsay, F-75321 Paris Cédex 07(FR)**

(72) Inventeur: **Dupont, René, 14, rue Saint Eloi, F-59500 Douai(FR)**
Inventeur: **Simonet, Guy, 105, Cours de Vincennes, F-75020 Paris(FR)**

(74) Mandataire: **Vesin, Jacques et al, L'AIR LIQUIDE, SOCIETE ANONYME POUR L'ETUDE ET L'EXPLOITATION DES PROCEDES GEORGES CLAUDE 75, quai d'Orsay, F-75321 Paris Cédex 07(FR)**

## Description

La présente invention concerne un procédé de reformage du méthanol impur et un dispositif de mise en oeuvre.

La coupure du méthanol, soit par un procédé de craquage ou de vaporeformage, peut conduire à des mélanges de gaz de compositions nettement différentes; et aussi bien le choix approprié de catalyseurs que les conditions opératoires, déterminent en grande partie la composition du flux gazeux résultant.

L'emploi du méthanol comme source de production d'hydrogène, bien que très connu, était considéré comme présentant des désavantages évidents notamment au point de vue économique. Cependant, la disponibilité décroissante des hydrocarbures, ainsi que la gazéification du charbon tendant à changer cette situation.

Les techniques existantes ont déjà été améliorées et adaptées, afin de réaliser un emploi optimal du méthanol comme hydrocarbure de départ pour la fabrication de gaz à compositions variées.

Pour la production d'hydrogène, la technique du vaporeformage semble particulièrement bien adaptée, une partie de l'eau introduite étant transformée en hydrogène; et dans des conditions de mise en oeuvre à basse température il convient d'avoir recours à un catalyseur dépourvu d'activité vis à vis de la méthanisation.

Par le brevet français 2.490.615, on connait un procédé de reformage du méthanol en vue de l'obtention de gaz contenant des concentrations variables d'hydrogène, d'oxyde de carbone, le solde du gaz étant composé de dioxyde de carbone, de méthane, de vapeur d'eau et éventuellement d'azote, selon lequel on procède à une opération de craquage de mélanges en proportions variables de vapeur d'eau et de vapeur de méthanol, en présence éventuellement d'azote ou d'air et de gaz recyclés, après séparation éventuelle de certains composants et de gaz en provenance de l'extérieur de l'unité. Ce procédé mis en oeuvre en plusieurs étapes permet de produire de l'hydrogène pur.

Dans l'étape a) on réalise le chauffage et la vaporisation à la pression requise, par exemple 20 bars, du mélange d'alimentation ajusté méthanol-eau.

Puis dans l'étape b) le reformage sur catalyseur adapté du mélange méthanol-eau est conduit à une température de 300°C environ

Cette opération s'effectue suivant deux réactions simultanées en équilibre, dont l'ensemble constitue le reformage :

Décomposition du méthanol : $CH_3OH \rightleftharpoons CO + 2H_2$
Conversion du monoxyde de carbone : $CO + H_2O \rightleftharpoons CO_2 + H_2$

Ces deux réactions peuvent être conduites soit sur un seul catalyseur, soit sur deux catalyseurs.

Le gaz obtenu en sortie du reformage, à 300°C, a la composition approchée suivante, exprimée en pourcentage molaire, pour un rapport $CH_3OH/H_2O = 0,5$ à l'entrée du réacteur : hydrogène $H_2 = 55,2$ % environ, dioxyde de carbone $CO_2 = 17,8$ % environ, monoxyde de carbone $CO = 0,9$ % environ, eau $H_2O = 26,1$ % environ, avec des traces de méthane ou de méthanol.

Dans l'étape c) on procède au refroidissement du gaz reformé jusqu'à la température ambiante avec condensation d'eau.

Après le refroidissement, sous 20 bars par exemple, la composition du mélange est la suivante : $H_2 = 74,3$ % environ, $CO_2 = 24,0$ % environ, $CO = 1,2$ % environ, $H_2O = 0,5$ % environ, avec des traces de méthane et de méthanol.

Ensuite dans une dernière étape d) on réalise la purification du mélange gazeux sur tamis moléculaire. Ce procédé est avantageux en combinaison avec le vaporeformage du méthanol, notamment en vue de la production d'un hydrogène très pur.

La purification du mélange gazeux sus la pression précitée par adsorption des gaz autres que l'hydrogène dans une unité du type "Pressure swing adsorption" PSA, permet d'obtenir, d'une part, une fraction d'hydrogène pur sous pression, constituant le produit de l'unité de reformage, et d'autre part, une fraction dite "gaz résiduaire", disponible à une pression réduite et dont la composition type exprimée en volume, est la suivante : $H_2 = 47$ % environ, $CO_2 = 48,2$ % environ, $CO = 3,4$ % environ, $H_2O = 1,2$ % environ, $CH_4 = 0,2$ % environ.

Selon le procédé traitant du méthanol pratiquement pur, l'eau condensée lors de la phase c) est recyclée à l'entrée de l'unité. La chaleur nécessaire au reformage est généralement obtenue par chauffage d'un fluide caloporteur 320°C environ, dans une zone de combustion, où la fraction de gaz résiduaire est brûlée. Ce fluide caloporteur fait ensuite l'objet d'un échange thermique avec la zone de reformage catalytique, les tubes de catalyseur d'une part, - reformage de type isotherme - et avec le mélange gazeur à reformer d'autre part - pour assurer notamment sa vaporisation.

Le procédé précédemment décrit conduit à de très bons résul tats à partir de méthanol pratiquement pur. Dans le cas où le méthanol contient un certain nombre d'impuretés constituées par des alcools plus lourds que le méthanol, et si l'on maintient les conditions habituelles de fonctionnement du catalyseur, c'est-à-dire à une température de 300°C environ, les impuretés du méthanol ne réagissent que très peu sur le catalyseur, d'une part, et réduisent le rendement du reformage du méthanol d'autre part.

Il en résulte un enrichissement de l'eau condensée, dans la phase c), en méthanol et en alcools divers. Cet enrichissement est en relation avec le rapport eau/méthanol du mélange gazeux entrant sur le catalyseur. Le tableau ci-dessous donne quelques valeurs expérimentales

| Composition du liquide condensé (% poids) | Rapport $H_2O/CH_3OH$ | |
|---|---|---|
| | 1,75 | 1,5 |
| Eau | 80,5 | 73,45 |
| Méthanol | 15,6 | 22,00 |
| Ethanol | 1,15 | 1,35 |
| Isopropanol | 0,80 | 0,90 |
| Propanol | 1,05 | 1,20 |
| Butanol | 0,90 | 1,10 |

Il a été recherché un procédé perfectionné de reformage permettant de traiter du méthanol contenant des impuretés constituées principalement par de l'éthanol, de l'isopropanol, du propanol, du butanol, des alcools gras divers, etc..., tout en palliant les inconvénients inhérents au procédé précédemment décrit.

Selon l'invention, dans un procédé de reformage de méthanol impur contenant des alcools supérieurs au méthanol, dans lequel l'apport de chaleur se fait par l'intermédiaire d'un fluide caloporteur chauffé par le gaz résiduaire d'une purification du gaz produit par reformage, en vue de l'obtention d'hydrogène, on réalise une combustion simultanée du mélange condensé eau-alcools provenant du refroidissement du gaz reformé jusqu'à l'ambiante, et du gaz résiduaire provenant de la purification de l'hydrogène.

La combustion simultanée du mélange condensé-vaporisé et du gaz résiduaire de l'épuration hydrogène, présente de nombreux avantages.

Elle permet l'incinération d'un liquide ne contenant qu'une teneur relativement réduite en méthanol et alcools divers, donc l'ob tention d'un meilleur rendement de reformage du méthanol impur.

Cette combustion simultanée évite toute zone particulière d'incinération du liquide, cette incinération s'effectuant dans la zone de combustion du gaz résiduaire, pour fournir la chaleur utile au reformage du méthanol.

En outre, la récupération de la chaleur propre à la combustion du flux liquide purgé peut se faire de façon économique nécessitant seulement un moyen supplémentaire simple de récupération thermique dans la zone de combustion du gaz résiduaire (production de vapeur, par exemple).

De plus, l'incinération du mélange liquide eau-alcools dans la zone de combustion du mélange résiduaire évite tout problème d'environnement lié au rejet d'un tel mélange liquide.

Selon les cas de fonctionnement, la combustion simultanée du liquide condensé dit purgé, eau-alcools et du gaz résiduaire d'épuration, permet la récupération d'une quantité de chaleur disponible en excédent des besoins propres au procédé lui-même.

Suivant une variante du procédé, on introduit d'autres liquides vaporisables dans le liquide purgé eau-alcools, et le mélange après vaporisation est soumis à une combustion simultanée avec le gaz résiduaire d'épuration. Cette condition opératoire permet d'accroître la récupération de la chaleur.

On peut avantageusement mettre en oeuvre en combinaison, la combustion mixte du mélange condensé eau-alcools et du gaz résiduaire d'épuration avec le recyclage dans la zone de reformage d'une fraction dudit mélange condensé, ladite fraction recyclée étant ajoutée au flux d'alimentation : le méthanol et l'eau, avant vaporisation par échange thermique avec le gaz sortant du réacteur. Selon les cas on peut obtenir une autonomie thermique du procédé sans excédent de chaleur disponible.

Le rapport des proportions entre la fraction de mélange condensé eau-alcools envoyé à la combustion et la fraction de mélange recyclé au reformage, est régulé en fonction des impuretés du méthanol à traiter, de manière à obtenir le meilleur compromis possible entre les deux facteurs économiques importants du procédé, à savoir le rendement de reformage du méthanol et la durée de vie du catalyseur, directement lié à la température de reformage.

La température de reformage est en général d'autant plus élevée que la fraction de liquide condensé recyclé est plus importante ; les conditions de travail du catalyseur étant alors plus sévères.

Un avantage important de la présente invention est constitué par le maintien de la température de réaction de reformage à 300°C environ, température très inférieure à celle qui serait nécessaire pour traiter un méthanol impur dans le procédé adiabatique décrit dans le premier certificat d'addition FR 2.572.380.

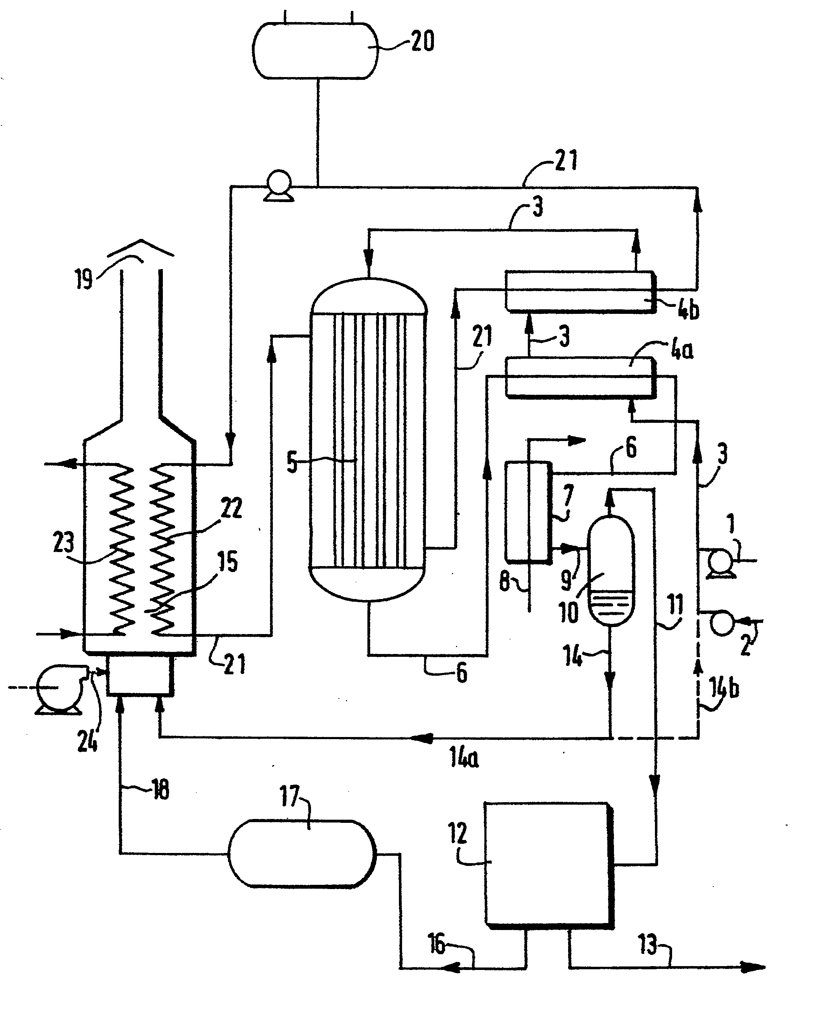

Le vaporeformage du méthanol impur peut être conduit dans une installation du type représenté sur la figure annexée.

Cette installation comprend essentiellement un réacteur tubulaire, dans lequel s'effectue le vaporeformage, un vaporiseur du mélange réactionnel, et un échangeur de chaleur entre les gaz sortants et le flux d'alimentation, un condenseur, une unité de purification, en vue de l'obtention d'hydrogène pur, un four de combustion équipé de deux faisceaux tubulaires et un circuit de recyclage.

Les liquides d'alimentation, le méthanol et l'eau introduits en (1) et (2) après mélange et circulation dans la conduite (3), sont chauffés et partiellement évaporés dans l'échangeur de chaleur (4a) par échange thermique avec le gaz sortant du réacteur (5) et circulant dans la canalisation (6), et le mélange réactionnel est préchauffé dans le vaporiseur (4b). Après vaporisation et chauffage à la température requise le mélange réactionnel circule dans les tubes chargés de catalyseur du réacteur (5) où s'effectue les réactions de reformage du méthanol et d'équilibre $CO + H_2O \leftrightarrows CO_2 + H_2$. Le mélange gazeux résultant de ces réactions et sortant du réacteur (gaz humide) est d'abord refroidi dans l'échangeur (4a) en contre-courant du liquide à traiter.

Ledit gaz humide sortant de l'échangeur (4a) est ensuite refroidi dans le refroidisseur-condenseur (7) à circulation d'eau (8) avant d'entrer par (9) dans le séparateur (10), puis le mélange gazeux quitte la partie supérieure du séparateur par la canalisation (11) avant d'entrer dans l'unité de purification (12), l'hydrogène pur quittant l'installation en (13). Le liquide mélange eau-alcools condensé est soutiré du séparateur (10) vers la canalisation (14) avant d'être introduit dans le four de combustion (15). Le gaz résiduaire quitte l'installation de purification par le conduit (16), est accumulé dans le réservoir-tampon (17) avant de circuler par (18) jusqu'au four de combustion (15) pour y être brûlé simultanément avec le mélange eau-alcools, les fumées s'échappent en (19), tandis que la chaleur est récupérée d'une part par un fluide caloporteur provenant du réservoir (20) et circulant dans la boucle (21), entre le four de combustion (15) dans le dispositif d'échange thermique (22), situé dans ledit four, le réacteur (5) et le vaporiseur (4b) et d'autre part, dans le dispositif d'échange thermique supplémentaire (23) également situé dans le four de combustion (15). L'air ou l'oxygène sont amenés au brûleur du four, après compression, par la canalisation (24).

Selon une variante, le mélange condensé, eau-alcools, soutiré par la canalisation (14) est séparé en deux fractions, dans ce cas une fraction (14a) est comme précédemment introduite dans le four de combustion (15) et la fraction (14b) est recyclée dans le mélange d'alimentation méthanol-eau dans la conduite (3).

Selon une autre variante, non représentée, dans le cas où le méthanol contient des impuretés lourdes, l'installation classique comportant une pompe-méthanol, et une pompe eau, le mélange se faisant au refoulement de pompe est remplacé par le dispositif suivant :
- mélange eau + méthanol impur dans un réservoir à la pression atmosphérique (selon la proportion requise par le procédé) ;
- filtration du mélange après un temps approprié (de quelques heures à quelques dizaines d'heures) sur un filtre spécial pour séparer la phase solide s'étant constituée dans le réservoir précité.

Le liquide filtré est recueilli dans un second réservoir à la pression atmosphérique à partir duquel une pompe alimente l'unité de reformage à la pression désirée.

Les moyens précédemment décrits ont été mis en oeuvre dans une unité prototype.

Et les résultats obtenus sont consignés dans le tableau ci-dessous.

| Teneur en Impuretés lourdes dans $CH_3$ OH traité | Taux de recyclage du condensat liquide | Température du reformage | Rendement $H_2$ Produit au reformage par kg de $CH_3OH$ consommé |
|---|---|---|---|
| % poids | % | °C | $Nm^3 H_2$ / kg |
| 2,45 | 0 | 284 | 1,79 |
| 5,40 | 57 | 287 | 1,88 |
| 8,50 | 75 | 291 | 1,92 |
| 9,70 | 85 | 293 | 1,95 |

**Revendications**

1. Procédé de reformage de méthanol impur, contenant des alcools supérieurs au méthanol, dans lequel l'apport de chaleur se fait par l'intermédiaire d'un fluide caloporteur chauffé par le gaz résiduaire, d'une purification du gaz produit par reformage, en vue de l'obtention d'hydrogène pur, caractérisé en ce qu'on réalise une combustion simultanée du mélange condensé eau-alcools provenant du refroidissement du gaz reformé, d'une part, et du gaz résiduaire provenant de la purification de l'hydrogène.

2. Procédé de reformage de méthanol impur, selon la revendication 1, caractérisé en ce que l'on introduit dans le mélange condensé eau-alcools d'autres liquides vaporisables.

3. Procédé de reformage de méthanol impur selon la revendication 1 ou 2, caractérisé en ce qu'on met en oeuvre en combinaison, la combustion mixte du mélange condensé eau-alcools et du gaz résiduaire d'épuration avec le recyclage au reformage d'une fraction du dit mélange condensé; la dite fraction recyclée étant ajoutée au flux d'alimentation, méthanol-eau, avant vaporisation.

4. Procédé de reformage de méthanol impur, selon la revendication 3, caractérisé en ce que le rapport des proportions entre la fraction envoyée à la combustion et la fraction recyclée est régulé en fonction des impuretés du méthanol à traiter.

5. Installation de reformage de méthanol impur pour la mise en oeuvre du procédé selon les revendications 1 ou 2, comprenant un réacteur tubulaire (5), dans lequel s'effectue le vaporeformage, le vaporiseur (4b) du mélange réactionnel, l'échangeur de chaleur (4a) entre les gaz sortants et le liquide d'alimentation, un séparateur (10), une unité de purification (12),en vue de l'obtention d'hydrogène pur, et un four de combustion (15) du gaz résiduaire d'épuration, caractérisé en ce que l'installation comprend en outre une canalisation (14) d'amenée du mélange condensé eau-alcools au four de combustion (15) équipé de deux dispositifs d'échange thermique (22) et (23).

6. Installation de reformage de méthanol impur selon la revendication 5, caractérisée en ce qu'elle comporte en outre une canalisation (14 b) de recyclage du mélange condensé eau-alcools aboutissant dans la canalisation (3) d'alimentation.

7. Installation de reformage de méthanol impur selon la revendication 5, caractérisée par un réservoir de mélange eau-méthanol, un filtre de séparation de la phase solide, un réservoir du liquide filtré, à partir duquel une pompe alimente l'unité de reformage.

**Claims**

1. A method of reforming impure methanol containing alcohols higher than methanol, in which the input of heat occurs via a heat-carrier fluid heated by the residual gas from a purification of the gas produced by reforming for the purpose of obtaining pure hydrogen, characterised in that a simultaneous combustion is effected of the condensed mixture of water and alcohols coming from the cooling of the reformed gas, on the one hand, and of the residual gas coming from the purification of hydrogen.

2. A method of reforming impure methanol according to claim 1, characterised in that other vaporisable liquids are introduced into the condensed water/alcohol mixture.

3. A method of reforming impure methanol according to claim 1 or 2, characterised in that use is made in combination, of the mixed combustion of the condensed water/alcohol mixture and of the residual purification gas with the recycling to the reforming operation of a fraction of the said condensed mixture; the said recycled fraction being added to the methanol-water feed flow prior to vaporisation.

4. A method of reforming impure methanol according to claim 3, characterised in that the ratio of the proportions between the fraction conveyed to the combustion and the recycled fraction is governed as a function of the impurities of the methanol to be processed.

5. Apparatus for reforming impure methanol, for application of the method according to claim 1 or 2, comprising a tubular reactor (5) wherein the steam reforming takes place, the evaporator (4b) for the reactive mixture, the heat exchanger (4a) between the outgoing gases and the feed liquid, a separator (10), a purification unit (12), intended to obtain pure hydrogen, and a furnace (15) for combustion of the residual purification gas, characterised in that the apparatus furthermore comprises a pipe (14) for bringing in the condensed water/alcohol mixture into the combustion furnace (15) provided with two heat exchange devices (22) and (23).

6. Apparatus for reforming impure methanol according to claim 5, characterised in that it also comprises a pipe (14b) leading into the feed pipe (3) for recycling the condensed water/alcohol mixture.

7. Apparatus for reforming impure methanol according to claim 5, characterised by a tank of water-methanol mixture, a filter for separation of the solid phase, a tank of filtered liquid from which a pump supplies the reforming unit.

**Patentansprüche**

1. Verfahren zur Reformierung von unreinem Methanol mit einem Gehalt höherer Alkohole als Methanol, bei dem die Wärmezufuhr mit Hilfe eines wärmeführenden Fluids erfolgt, das durch das Restgas einer Reinigung des durch Reformierung zur Gewinnung von reinem Wasserstoff erzeugten Gases erwärmt wurde, dadurch gekennzeichnet, daß man eine gleichzeitige Verbrennung einerseits des aus der Abkühlung des reformierten Gases stammenden kondensierten Wasser-Alkohol-Gemisches und andererseits des aus der Reinigung des Wasserstoffs stammenden Restgases durchführt.

2. Verfahren zur Reformierung von unreinem Methanol nach Anspruch 1, dadurch gekennzeichnet, daß man in das kondensierte Wasser-Alkohol-Gemisch andere verdampfbare Flüssigkeiten einführt.

3. Verfahren zur Reformierung von unreinem Methanol nach Anspruch 1 oder 2, dadurch gekennzeichnet, daß man in Kombination die gemischte Verbrennung des kondensierten Wasser-Alkohol-Gemisches und des Restgases der Reinigung mit der Rückführung eines Anteils dieses kondensierten Gemisches zur Reformierung durchführt, wobei dieser rückgeführte Anteil dem Beschickungsstrom von Methanol-Wasser vor der Verdampfung zugesetzt wird.

4. Verfahren zur Reformierung von unreinem Methanol nach Anspruch 3, dadurch gekennzeichnet, daß das Verhältnis zwischen dem der Verbrennung zugeführten Anteil und dem zurückgeführten Anteil in Funktion der Verunreinigungen des zu behandelnden Methanols gesteuert wird.

5. Vorrichtung zur Reformierung von unreinem Methanol nach dem Verfahren gemäß den Ansprüchen 1 oder 2 mit einem Röhrenreaktor (5), in welchem die Dampfreformierung erfolgt, dem Verdampfer (4b) für das Reaktionsgemisch, dem Wärmeaustauscher (4a) zum Wärmeaustausch zwischen den austretenden Gasen und der Beschickungsflüssigkeit, einer Trennvorrichtung (10), einer Reinigungsanlage (12) zur Gewinnung von reinem Wasserstoff und einem Verbrennungsofen (15) für Restgas der Reinigung, dadurch gekennzeichnet, daß die Vorrichtung außerdem eine Leitung (14) für die Zuführung des kondensierten Wasser-Alkohol-Gemisches zu dem Verbrennungsofen (15) aufweist, welcher mit zwei Wärmetauscheinrichtungen (22) und (23) ausgestattet ist.

6. Vorrichtung zur Reformierung von unreinem Methanol nach Anspruch 5, dadurch gekennzeichnet, daß sie außerdem eine Leitung (14b) zur Rückführung des kondensierten WasserAlkohol-Gemisches aufweist, das in der Beschickungsleitung (3) resultiert.

7. Vorrichtung zur Reformierung von unreinem Methanol nach Anspruch 5, gekennzeichnet durch einen Vorratsbehälter für Wasser-Methanol-Gemisch, ein Filter zur Abtrennung der festen Phase und einen Vorratsbehälter für filtrierte Flüssigkeit, von welcher eine Pumpe die Reformierungseinheit speist.